Europäisches Patentamt

European Patent Office

Office européen des brevets

(11) Publication number: **0 129 807**

**A2**

## (12) EUROPEAN PATENT APPLICATION

(21) Application number: 84106896.8

(22) Date of filing: 15.06.84

(51) Int. Cl.⁴: **A 61 K 7/09**

(30) Priority: 21.06.83 JP 111661/83

(43) Date of publication of application:
02.01.85 Bulletin 85/1

(84) Designated Contracting States:
AT CH DE FR GB LI

(71) Applicant: Kao Corporation
14-10, Nihonbashi Kayabacho 1 chome
Chuo-Ku Tokyo 103(JP)

(72) Inventor: Naito, Sachio
4-35-12-303, Kamiyoga Setagaya-ku
Tokyo(JP)

(72) Inventor: Mikata, Tsuruo
1875-278, Nakahara
Kashiwa-shi Chiba-ken(JP)

(72) Inventor: Okumura, Takeo
2-7-7, Takinoi
Funabashi-shi Chiba-ken(JP)

(74) Representative: Wächtershäuser, Günter, Dr.
Tal 29
D-8000 München 2(DE)

(54) Hair treatment composition for heat waving and method for waving hair.

(57) A hair treatment composition for heat waving comprising at least one compound selected from the group consisting of an amino acid free of a mercapto group, peptide, protein, and derivatives thereof.

With a composition according to the invention, hair can be firmly waved by a simple operation under a relatively low temperature and in short time.

Since the composition does not include neither alkaline solutions of high concentrations nor reducing agents such as mercapto compounds the damage of hair caused by elution of hair proteins can be mitigated.

EP 0 129 807 A2

## BACKGROUND OF THE INVENTION

i)    Field of the Invention:

This invention relates to compositions for heat hair waving treatment and a method for waving hair with such composition.  More particularly, it relates to hair treatment compositions for heat waving which make no use of reducing substances such as mercapto compounds, sulfites and the like as is used in known permanent wave solutions but are able to have hair semi-permanently waved only by application of heat.  Also, it relates to a method for waving hair utilizing the compositions.

ii)    Description of the Prior Art:

A typical method of having hair permanently waved in a desired degree was initially a method called "heat waving" in which hair is applied with an aqueous alkali solution of high concentration (high pH) and heated.  The most popular method in recent years is a so-called cold permanent waving method.  In the method, a primary permanent waving solution comprising as its principal ingredient a reducing agent such as thioglycollic acid, cysteine, sulfite or the like is applied to hair in order to open the S-S bonds in the hair by reduction. Subsequently, a secondary permanent waving solution comprising an oxidizing agent such as a bromate, perborate, hydrogen peroxide or the like is applied to close the bonds by oxidation.

However, these methods have the disadvantages that the hair is subjected to severe conditions of the application of an alkali solution of high concentration and the heating to high temperatures, or the reduction and oxidation, causing the hair to be lowered in strength and to deteriorate in feeling to the touch. Because of the heating to high temperatures and the use of highly irritative chemicals, care should be paid from the standpoint of safety.

Quite recently, various methods of waving hair without use of any known techniques have been proposed including a method for permanently setting hair using vinyl polymers (United States Patent No. 3,248,376), a setting method using silicone films (United States Patent No. 2,782,790), and a setting method in which hair fibers are modified by lanthionine bonds (Japanese Laid-open Application No. 50-29756).

However, these methods involve a problem of safety on skin because of the use, as substrate, of acrylonitrile (United States Patent No. 3,248,376) and a

4

hydroxy compound of dimethylsiloxane and an alkyl derivative of titanic acid (United States Patent No. 2,782,790), and of the treatment with an alkali solution of high concentration. Thus, any of these methods have not be reduced into practice.

## SUMMARY OF THE INVENTION

We have made intensive studies on a method of waving hair and particularly a method capable of semi-permanently setting hair similar to the waved hair attained by known permanent waving techniques without use of alkalis of high concentration, and oxidizing and reducing agents which will damage hair. As a result, it has been found that when hair is applied with a hair treatment composition comprising a specific type of amino acid, peptide, protein or derivatives thereof and adjusted in pH to 8 to 10.5, and then heated to a predetermined temperature, the hair is semi-permanently set or waved as desired.

The hair treatment composition has a low concentration of an alkali and a low heating temperature is sufficient for the setting, so that high safety against skin is ensured.

5

Moreover, if a buffer agent is added to the treatment composition, better hair setting or waving effects can be obtained.

The present invention is accomplished based on the above findings.

According to one embodiment of the invention, there is provided a hair treatment composition for heat waving which comprises at least one compound selected from the group consisting of an amino acid free of a mercapto group peptide, protein, and derivatives thereof (hereinafter referred to simply as peptide compound(s)), the composition having a pH ranging from 8 to 10.5. The composition may further comprise a buffer agent.

According to another embodiment of the invention, there is provided a method of waving hair which comprises applying the treatment composition of the above-described type to hair and heating the hair to a temperature ranging from 40 to 160 °C

BRIEF DESCRIPTION OF THE DRAWINGS

The sole figure is a graphical representation of the relation between treating time and degree of waving in case where hair is treated with a permanent waving treatment of the invention and an ammoniacal solution with a pH of 10.

### DETAILED DESCRIPTION OF THE INVENTION
### AND PRFFERED EMBODIMENTS

Examples of the amino acids free of mercapto group useful in the present invention include neutral amino acids such as alanine, glycine, valine, isoleucine, leucine, proline, cystine, phenylalanine, serine, threonine, hydroxypyrroline, tyrosine, tryptophane and the like, basic amino acids such as arginine, lysine, histidine, and the like, acidic amino acids such as aspartic acid, glutamic acid and the like, and derivatives thereof. Of these, alanine, serine, threonine, cystine and lysine are preferable.

The peptides and proteins are (1) dimer or larger polypeptides artificially prepared from one or more amino acids indicated above, (2) naturally occurring proteins such as keratin, collagen, elastin, sericin, fibroin, albumin, globlin, conglutinine, casein, soybean protein and the like, and their hydrolyzates, decomposition products by oxidation, and derivatives or SH-modified compounds of decomposition products by reduction thereof, which are produced according to the technique described in Japanese Laid-Open application No. 57-85308, and (3) naturally occurring hormones and physiologically active peptides such as, for example, insulin, glutathione and the like. Examples of (1) include polypeptides prepared from one or more of basic acids such as lysine, arginine and the like, and acidic acids such as glutamic acid, aspartic acid and the like. Preferable peptides and proteins include simple proteins such as keratin, collagen, sericin, fibroin and the like, decomposition products by oxidation thereof, hydrolyzates thereof with enzyme, acid or alkali, polylysine, hydrolyzates of soybean proteins, and insulin.

The hair waving treatment of the invention (hereinafter referred to simply as treatment or treatment composition) is adjusted in pH with alkalis. No limitation is imposed on the type of alkali. Any alkalis which are able to adjust the pH to 8 to 10.5 may be used. Examples of the alkalis include alkali metal or alkaline earth metal hydroxides such as potassium hydroxide, sodium hydroxide, lithium hydroxide, calcium hydroxide and the like, ammonia, and organic alkalis such as monoethanolamine, diethanolamine, triisopropanolamine, 2-amino-2-methyl-1,3-propanediol,

2-amino-2-methyl-1-propanol and the like. Inorganic salts such as sodium carbonate, calcium carbonate, ammonium carbonate and the like which show alkalinity when dissolved in water may be also used.

In the treatment of the present invention, if a buffer agent is used for the pH adjustment, it is possible to reduce amounts of the peptide compounds. When no buffer agent is used in the treatment of the invention, at least one peptide compound has to be used, in a final usage form, in an amount of 0.1 to 20 wt% (hereinafter referred to simply as %), preferably from 0.5 to 10%, of the total composition. However, where a buffer agent is added, the amount of the at least one peptide compound is sufficient to range from 0.01 to 10% preferably 0.1 to 5%.

The buffer agents used in the practice of the invention include, for example, citric acid/disodium hydrogenphosphate, hydrochloric acid/sodium barbital/sodium acetate, hydrochloric acid or maleic acid/trishydroxyaminomethane, potassium or sodium dihydrogenphosphate/dipotassium or sodium hydrogenphosphate, hydrochloric acid or potassium or sodium dihydrogenphosphate/sodium tetraborate, potassium or sodium dihydrogenphosphate/sodium or potassium hydroxide, hydrochloric acid/collidine, boric
acid/sodium carbonate or sodium tetraborate,
hydrochloric acid/aminomethylpropanediol, glycine/sodium
or potassium hydroxide, boric acid/sodium hydroxide,
hydrochloric acid/sodium methylglycine, sodium
hydrogencarbonate/sodium carbonate, sodium
tetraborate/sodium hydroxide, sodium
hydrogencarbonate/sodium hydroxide, water-soluble
ammonium salt/ammonia, and the like. Of these buffer
agents, a combination of water-soluble ammonia
salt/ammonia is preferred because they are volatile,
scarcely remain on hair and skin, and cause only a small
degree of damage on hair and irritativeness on skin.
Counter ions for the ammonium are preferably chlorine,
carbonate and bicarbonate ions.

Where a buffer agent is added to the treatment
of the invention, its total amount is from 0.05 to 10%,
preferably from 0.1 to 5%, of the total composition.
When, for example, a water-soluble ammonium salt/ammonia
buffer agent is added, the water-soluble ammonium salt
such as ammonium bicarbonate, ammonium carbonate,
ammonium chloride or the like is first added, followed
by adding ammonia until a pH of from 8 to 10.5 is
reached.

Aside from the above-described essential components, the treatment of the invention may further comprise other ingredients ordinarily used for these purposes, including suitable oils, penetrants, humectants, colorants, emulsifiers, perfumes and the like.

The treatment obtained in this manner is applied as follows. First, the treatment is applied to hair. Prior to the application of the treatment, it is preferred that the hair is wound about a rod or the like to be waved as desired. Although the amount of the treatment depends on the treating conditions including heating temperature, it is preferably from 10 to 150 ml in one application. Then, the hair is heated to a temperature of 40 to 160°C. The heating temperature and time are determined in consideration of the degree of damage of hair, the type of pepetide, the types of buffer agent and alkali, the pH and the type of preparation of the treatment. Higher temperatures are more advantageously applied to healthy hair which is not permed, dyed or bleached. In view of the fact that hair is damaged by application of heat, the temperature is from 40 to 160°C, preferably from 40 to 80°C. Needless to say, lower temperatures require a longer time. For the same reason as mentioned with respect to the temperature, the heating time is below 30 minutes, preferably from 3 to 10 minutes. With chemically treated hair, e.g. permed, dyed or bleached hair, it is desirable to use milder conditions.

According to the invention, hair can be firmly waved by a simple operation under relatively low temperature and short time conditions. Neither alkaline solutions of high concentrations nor reducing and oxidizing agents are used, the damage of hair caused by elution of hair proteins can be mitigated with a reduced degree of irritativeness against skin. The treatment of the invention has good storage stability and has thus the advantage that problems involved in handling of known permanent waving solutions can be solved.

The present invention is described in more detail by way of examples, which should not be construed as limiting the invention.

Example 1

Treatments (aqueous solutions) of the formulations indicated in Table 1 were tested to determine a degree of waving and a wave holding capability or property. The results are shown in Table 1.

[Measuring Method]

Test of Measuring a Degree of Waving and a
Wave Holding Force

(i)  Twenty hair fibers were combined to give
a bundle and were set to pins, each having a diameter of
2 mm and a length of 1.5 cm, of a wave measuring plate
which were arranged in two rows with a zigzag form.  The
plate was immersed in the respective permanent waving
solutions at 80°C for 30 minutes.  After rinsing
sufficiently with water, the bundle was removed from the
measuring plate and length of the hair bundle was
measured in still water.  A degree of waving was
calculated according to the following equation.

The hair fibers used were 20 cm long virgin
hair fibers which were washed with an aqueous solution
of 0.5% sodium laurylsulfate and dried.

$$\text{Degree of waving (\%)} = \frac{X - Z}{X - Y} \times 100$$

X:  Length of hair passed between adjacent
pins A and B of one row arranged in zigzag
form with a pin of the other row.

Y:  Linear distance between A and B.

Z:  Distance between two points of the waved
hair contacted with pins A and B when measured as waved in still water after
removal from the measuring plate.

(ii)  The hair bundles used in (i) were each
dried in air for one day, after which it was immersed in
an aqueous solution of 0.5% sodium laurylsulfate at 40°C
for 1 minute during which the bundle was slightly moved
for washing.  Thereafter, each bundle was rinsed
sufficiently and then placed in still water to measure
the length, Z.  The degree of waving was determined
similar to (i) and a ratio of the degree of waving after
the washing to the degree of waving prior to the washing
was calculated to give a wave holding rate.

Waving holding rate (%) =

$$\frac{\text{Degree of Waving after washing}}{\text{Degree of waving prior to washing}} \times 100$$

Table 1

| Treatment Composition | | | | Degree of Waving | Wave Holding Rate (%) |
|---|---|---|---|---|---|
| Peptide Compound | Conc. (%) | Alkali Agent | pH | | |

**Product Invention**

| | | | | | | |
|---|---|---|---|---|---|---|
| 1 | L-alanine | 0.1 | ammonia | 10 | 40 | 34 |
| 2 | " | 5.0 | ammonia | 10 | 48 | 41 |
| 3 | DL-alanyl-DL-alanine | 5.0 | ammonia | 10 | 52 | 44 |
| 4 | L-glycyl-L-glycine | 5.0 | ammonia | 10 | 47 | 40 |
| 5 | Hydrolyzate of sericin and fibroin with acid (M.W. 350) | 5.0 | ammonia | 8 | 34 | 29 |
| 6 | " | 5.0 | ammonia | 9 | 52 | 44 |
| 7 | " | 5.0 | ammonia | 10 | 61 | 52 |
| 8 | Hydrolyzate of keratin with acid (M.W. 1300) | 0.1 | ammonia | 10 | 42 | 36 |
| 9 | " | 5.0 | ammonia | 10 | 55 | 47 |
| 10 | Decomposition product of collagen with enzyme (M.W. 2000) | 5.0 | ammonia | 10 | 50 | 42 |
| 11 | " (M.W. 5000) | 5.0 | ammonia | 10 | 42 | 36 |
| 12 | Decomposition product of keratin by oxidation (alpha-keratose) (M.W. 150,000) | 5.0 | ammonia | 10 | 50 | 43 |
| 13 | Collagen (M.W. 100,000) | 5.0 | ammonia | 10 | 40 | 34 |

15

| Treatment Composition | | | | Degree of Waving | Wave Holding Rate (%) |
|---|---|---|---|---|---|
| Peptide Compound | Conc. (%) | Alkali Agent | pH | | |
| **Product Invention** | | | | | |
| 14 Hydrolyzate of collagen with enzyme (M.W. 2000) | 5.0 | sodium hydroxide | 10 | 45 | 38 |
| 15 " | 5.0 | mono-ethanolamine | 10 | 44 | 37 |
| **Comparative Product** | | | | | |
| – | – | ammonia | 10 | 26 | 10 |
| **Prior Art Cold Waving Treatment\*** | | | | | |
| – | – | – | – | 50 | 43 |

\*   Prior art cold waving treatment

Chemicals:

(1)   Primary cold waving solution (Reducer)

Ammonium thioglycollate                         7.0 (%)

Water, ammoniacal solution

(pH adjustment)                                 93.0

(pH adjusted to 9.0 with ammoniacal

solution)

(2)   Secondary cold waving solution (Neutralizer)

  Sodium bromate                              5.0 (%)

  Water                                       95.0


Treating Conditions:

    Immersed in the primary solution of 30°C for 10 minutes and then in the secondary solution of 30°C for 10 minutes.


**Example 2**

    A treatment of the invention having the following formulation was applied to healthy hair of Japanese, followed by heating at 80°C to determine a degree of waving as a function of time. The results are shown in a sole figure.

    For comparison, the above procedure was repeated using an ammoniacal solution.


[Composition]

|  | Treatment of Invention | Control |
|---|---|---|
| Hydrolyzates of sericin and fibroin with acid (M.W. 350) | 5(%) | — |
| Ammoniacal solution with a pH of 10 | 95 | 100 |

As will be clearly seen from the figure, the degree of waving reaches about 40% when the hair is treated at 80°C for 3 minutes, ensuring firm waving.

Example 3

A treatment of the invention which was made up of an aqueous solution containing 5% of a decomposition product (M.W. 2000) obtained by hydrolysis of collagen with enzyme and having a pH of 10 (adjusted with sodium hydroxide) was applied to healthy hair (Japanese) and heated at different temperatures for 30 minutes. The respective hairs were subjected to measurement of a degree of waving. The results are shown in Table 2 below.

Table 2

| Heating Temperature (°C) Formulation | 30 | 40 | 50 | 60 | 70 | 80 |
|---|---|---|---|---|---|---|
| Degree of Waving (%) — Treatment of Invention | 16 | 21 | 27 | 32 | 43 | 50 |
| Degree of Waving (%) — Only aqueous solution of sodium hydroxide with pH 10 | 14 | 15 | 18 | 22 | 24 | 26 |

It was confirmed that hair was satisfactorily waved when heated at temperatures of 40°C or higher.

**Example 4**

A treatment of the invention which was made up of an aqueous solution containing 5% of collagen (M.W. about 100,000) and having a pH of 10 (adjusted with ammoniacal solution) was applied to healthy hair (Japanese), followed by heating at 80°C for 30 minutes and rinsing sufficiently. The hair fibers were observed under a scanning electron microscope to judge the presence or absence of adsorbate on the fiber surfaces. The degree of adsorption was evaluated as follows, with the results shown in Table 3.

| Degree of Adsorption | Surface State of Hair Fibers |
| --- | --- |
| ++ | covered with film |
| + | adsorbate observed in very small amounts |
| - | no adsorbate observed |

For control, hair fibers which were treated in the same manner as in Example 1 using a setting lotion and a hair spray, both being used to transiently set hair and having compositions indicated below, and a known permanent waving treatment.

(Setting Lotion)

| | |
|---|---|
| Acrylic resin alkanolamine | 5.0 wt% |
| Ethanol | 50.0 |
| Ion-exchanged water | balance |

(Hair Spray)

| | |
|---|---|
| Methacrylate copolymer | 10.0 wt% |
| Ethanol | 29.9 |
| Propellant | 60.0 |
| Perfume | 0.12 |

[Results]

Table 3

| Treatment | Surface Condition of Hair |
|---|---|
| Treatment of Invention | − |
| Set Lotion | ++ |
| Hair Spray | ++ |
| Known Cold Waving Treatment | − |

The above results reveal that the waving of the hair according to the invention is not brought about by a transient setting effect produced by formation of polymer films on the surfaces of hair fibers.


**Example 4**

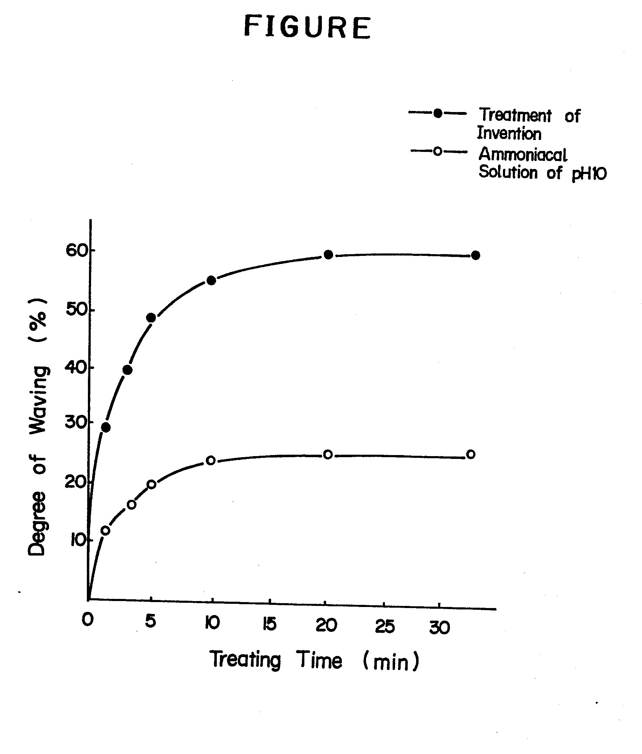

Treatments (0.4% ammonium chloride/ammonia buffer solution, pH 10) having the formulations indicated in Table 4 were tested to determine a degree of waving and a wave holding rate. The results are also shown in Fig. 4.


[Measuring Method]

Degree of waving and wave holding capability:

(i) Ten healthy hair fibers of Japanese with a length of 15 cm were combined to give a bundle and were wound around a glass tube having a diameter of 10 mm. The glass tube was immersed in the respective treatments at 80°C for 20 minutes, followed by rinsing sufficiently with water and removing from the glass tube. The curled hair was obtained. The length of the curled hair coil was measured. The degree of waving was calculated according to the following equation.

$$\text{Degree of waving (\%)} = \frac{Y}{X - Y} \times 100$$

X:  overall length of hair (15 cm)

Y:  length of the hair coil (cm)

(ii) The curled hair bundles used in (i) were air-dried for one day while suspending and immersed in an aqueous solution of 0.5% sodium laurylsulfate in which they are washed while gently moving. Subsequently, each bundle was rinsed sufficiently and the length of the hair coil was measured. The wave holding capability was evaluated as a wave holding rate calculated according to the following equation.

Wave holding rate (%) =

$$\frac{\text{degree of waving after washing}}{\text{degree of waving immediately after the treatment}} \times 100$$

0129807

## Table 4

| Treatment Composition | | | |
|---|---|---|---|
| Peptide compound | Conc. (%) | Degree of Waving (%) | Wave Holding Rate (%) |
| **Treatment of Invention** | | | |
| 1 Polyglutamic acid (M.W. over 10000) | 1.0 | 48 | 74 |
| 2 Polylysine (M.W. over 10000) | 1.0 | 53 | 81 |
| 3 " | 0.5 | 50 | 80 |
| 4 " | 0.1 | 48 | 73 |
| 5 Hydrolyzate of keratin protein (M.W. 630) | 1.0 | 47 | 78 |
| 6 Hydrolyzate of soybean protein (M.W. 1750) | 1.0 | 48 | 76 |
| 7 Insulin (M.W. 5320) | 1.0 | 46 | 81 |
| **Conventional Cold Waving Treatment*** | | | |
| – | – | 50 | 80 |

* Conventional cold waving treatment

Chemicals used and treating conditions same as used in Example 1.

23

## Example 5

A hair waving treatment prepared from a peptide indicated in Table 5 and a 3% ammonium chloride/ammonia buffer solution (pH 10) was applied to healthy hair (Japanese) and curled using a portable setting machine capable of heating and humidifying. The degree of waving at a heating temperature of 80°C was evaluated by a beauty artist as a function of time. The evaluation was made according to the following standard in comparison with the degree of waving in known cold waving treatment as a control. The results are shown in Table 5 below.

Evaluation Standard:

◎   Slightly higher

O   Same level

△   Slightly lower

X   Lower

Table 5

| Peptide | Treating Time (min.) | 1 | 3 | 5 | 10 | 20 |
|---|---|---|---|---|---|---|
| Invention Product | 5% Hydrolyzate of keratin (M.W. 610) | △ | O | O | O | ◎ |
| Control | — | X | X | X | △ | △ |

24

As will be clearly seen from the results of Table 5, the treatment of the invention is able to have hair waved to a degree equal to or better than in the case of the known cold waving treatment.

Example 6

Hair waving treatments of the following formulations were prepared and used to determine a degree of waving in the same manner as in Example 4 in which healthy hair fibers (Japanese) applied with such treatments were heated at different temperatures for 20 minutes. The results are shown in Table 6 below.

[Composition]

|  | Treatment of Invention | Control Treatment |
| --- | --- | --- |
| Polylysine | 1.0 (%) | – |
| 0.4% ammonium chloride/ ammonium buffer solution (pH 10) | 99 | 100 |

[Results]

Table 6

| Treatment | Heating Temperature (°C) | 30 | 40 | 50 | 60 | 70 | 80 |
|---|---|---|---|---|---|---|---|
| Degree of Waving (%) | Treatment of Invention | 19 | 28 | 34 | 40 | 48 | 52 |
| | Control Treatment | 17 | 22 | 28 | 32 | 38 | 43 |

From the above results, it will be seen that the hair is satisfactorily waved when the heating temperature is 40°C or higher.

**Example 7**

A treatment of the invention having the following formulation was prepared and applied to hair fibers, followed by observing the applied hair in the same manner as in Example 3. The results are shown in Table 7.

[Composition]

Polyglutamic acid (M.W. 10,000)          1 (%)

3% Ammonium chloride/ammonium

buffer solution (pH 10)                      99

- 25 -

[Results]

Table 7

| Treatment | Surface State of Hair |
|---|---|
| Treatment of Invention | − |
| Setting Lotion* | ++ |
| Hair Spray* | ++ |
| Known Cold Waving Treatment | − |

*   The compositions of the setting lotion and hair spray were same as those used in Example 3.

The above results demonstrate that the waving of the hair according to the invention is not caused by transient setting effects produced by formation of polymer films on the hair fibers.

0129807

WHAT IS CLAIMED IS:

1.   A hair treatment composition for heat waving comprising at least one compound selected from the group consisting of an amino acid free of a mercapto group, peptide, protein, and derivatives thereof, said composition having a pH ranging from 8 to 10.5.

2.   The hair treatment composition according to Claim 1, wherein said peptide is a dimer or polypeptide artificially synthesized from amino acids, or a derivative obtained by decomposition of proteins.

3.   The hair treatment composition according to Claim 1 or 2, wherein said peptide is a dimer or larger polypeptide synthesized from basic or acidic amino acids.

4.   The hair treatment composition according to Claim 1, wherein said polypeptide is a naturally occurring hormone or physiologically active peptide.

5.   The hair treatment composition according to Claim 1, wherein said peptide is a hydrolyzate of keratin or soybean protein.

6. A hair treatment composition for heat waving comprising at least one compound selected from the group consisting of an amino acid free of a mercapto group, peptide, protein and derivatives thereof, and a buffer agent, said composition having a pH ranging from 8 to 10.5.

7. The hair treatment composition according to Claim 6, wherein said peptide is a decomposition derivative of a dimer or larger polypeptide artificially prepared from amino acids, or of a protein.

8. The hair treatment composition according to Claim 6, wherein said peptide is a dimer or larger polypeptide prepared from one or more basic or acidic amino acids.

9. The hair treatment composition according to Claim 6, wherein said peptide is a naturally occurring hormone or physiologically active peptide.

10. The hair treatment composition according to Claim 6, wherein said peptide is a hydrolyzate of keratin or soybean protein.

11.  The hair treatment composition according to Claim 6, wherein said buffer agent is a member selected from combinations of citric acid/disodium hydrogenphosphate, hydrochloric acid/sodium barbital/sodium acetate, hydrochloric acid or maleic acid/trishydroxyaminomethane, potassium or sodium dihydrogenphosphate/dipotassium or sodium hydrogenphosphate, hydrochloric acid or potassium or sodium dihydrogenphosphate/sodium tetraborate, potassium or sodium dihydrogenphosphate/sodium or potassium hydroxide, hydrochloric acid/collidine, boric acid/sodium carbonate or sodium tetraborate, hydrochloric acid/aminomethylpropanediol, glycine/sodium or potassium hydroxide, boric acid/sodium hydroxide, hydrochloric acid/sodium methylglycine, sodium hydrogencarbonate/sodium carbonate, sodium tetraborate/sodium hydroxide, sodium hydrogencarbonate/sodium hydroxide, and water-soluble ammonium salt/ammonia.

12.  A method for waving hair comprising applying to hair a hair treatment composition for heat waving which comprises at least one compound selected from the group consisting of an amino acid free of mercapto group, peptide, protein, and derivatives thereof, said composition having a pH ranging from 8 to 10.5, and heating the applied hair to a temperature of from 40 to 160°C.

# FIGURE

—●— Treatment of Invention

—○— Ammoniacal Solution of pH10